# EUROPEAN PATENT APPLICATION

(11) **EP 4 215 490 A1**
(43) Date of publication of application: **26.07.2023**
(21) Application number: 21869054.3
(22) Date of filing: 02.08.2021
(51) Int. Cl.: C01G 23/00

(54) **AQUEOUS TITANIC ACID SOLUTION**

(30) Priority: 18.09.2020 JP 2020157100
(71) Applicant: Mitsui Mining & Smelting Co., Ltd., Shinagawa-ku Tokyo 141-8584 (JP)
(72) Inventor: HARA, Syuhei, Omuta-shi, Fukuoka 836-0003 (JP); KUMA, Taihei, Omuta-shi, Fukuoka 836-0003 (JP)
(74) Representative: Novagraaf Technologies
(86) International application number: PCT/JP2021/028629
(87) International publication number: WO 2022/059367

(57) **Abstract**

Provided is a novel titanate aqueous solution capable of easily preparing titanate compounds composed of alkali metals or alkaline earth metals, wherein the titanate aqueous solution contains titanate ions and quaternary ammonium cations in water, and the titanate aqueous solution is characterized in that 30 g of the titanate aqueous solution (25°C) adjusted to a concentration containing 9% by mass of titanium in terms of TiO₂ is added with 30 mL of a sodium hydroxide aqueous solution (25°C) having a concentration of 2.2% by mass while stirring to form a precipitate composed of Na₂Ti₃O₇ hydrate, or has a transmittance at a wavelength of 360 nm being 50% or less.

## Description

### TECHNICAL FIELD

The present invention relates to a titanate aqueous solution containing titanium or titanate in water.

### BACKGROUND ART

Titanium oxides are widely used in thin film applications such as photocatalytic materials, high refractive index materials, and conductive materials.

Titanate compounds composed of alkali metals or alkaline earth metals have various characteristics and are being used for various applications. For example, lithium titanate is used as an anode material for lithium secondary batteries and as an additive for ceramic insulators. Sodium titanate is used for various adsorbents, such as radioactive ion adsorbents. Potassium titanate has excellent sliding properties and wear resistance, and is thus used as an additive for imparting heat resistance, heat insulation, corrosion resistance, and reinforcement properties, in applications such as brake friction materials, precision gears, key switches, connectors, and bearings. Barium titanate is a ceramic exhibiting ferroelectricity, and is used for typical electronic component materials, such as capacitor materials, pyroelectric materials, and piezoelectric materials. Strontium titanate has high dielectric constant and small temperature change in normal dielectric constant, and is thus used in applications such as a material for ceramic capacitors.

Titanate aqueous solutions containing titanium or titanate in water can be widely used in applications such as surface treatment agents for various parts, raw materials for titanium oxide, and catalysts for esterification. The titanate aqueous solutions can also be used as raw materials or precursors for titanate compounds composed of alkali metals or alkaline earth metals, and the titanate compounds to be obtained can be expected to be effectively used for various industrial applications.

Aqueous solutions containing titanium or titanate are known to be obtained by reacting titanium alkoxide with a chelating agent. For example, Patent Literature 1 describes a method for producing a titanium-containing aqueous solution using an aliphatic amine.

As a compound to enhance stability in water, titanium chelate compounds using water-soluble substituents such as triethanolamine and lactic acid, and compounds obtained by reacting or mixing titanium alkoxide with glycol or amine are also known.

For example, Patent Literature 2 discloses an aqueous solution for forming a titanium oxide film containing titanium ions, nitrate ions, peroxide, and a complexing agent, and having a pH value of more than 3.0.

Patent Literature 3 discloses a water-soluble titanium oligomer composition that contains a titanium composite composition having a chemical structure and composition in which at least a titanium compound oligomer (a), an amine compound (b), and a glycol compound (c) are reacted and/or mixed.

Patent Literature 4 discloses an alkaline rutile-type titanium oxide sol having an average dispersed particle diameter of 15 to 70 nm and containing one or more compounds selected from water-soluble amine compounds in a molar ratio of 0.005 to 0.5 relative to a rutile-type titanium oxide (TiO₂).

Patent Literature 5 discloses a barium titanate precursor aqueous solution that can be prepared by mixing a water-soluble peroxohydroxycarboxylic acid titanium complex with a water-soluble barium salt, as a barium titanate precursor that forms barium titanate by calcination.

The following methods are also known as methods for preparing titanate compounds composed of alkali metals or alkaline earth metals. For example, a method of reacting a hydrated potassium titanate powder or titanium dioxide hydrate with a solution containing divalent metal ions in a sealed container or under hydrothermal conditions (Patent Literature 6); a method of reacting a crystalline fiber of potassium dititanate with an aqueous solution of a divalent metal compound (Patent Literature 7); a method of mixing an alkali metal titanate salt such as potassium dititanate or potassium hexatitanate serving as a titanium source with an inorganic or organic compound composed of an alkaline earth metal and a flux component (such as a halide of an alkali metal), followed by calcining the mixture (Patent Literature 8); and a method of heat-treating a mixture of a titanium oxide powder and a lithium compound powder at a temperature of 700°C to 1 ,600°C to obtain lithium titanate (Patent Literature 9), are known.

Furthermore, a method for preparing a titanium-containing aqueous solution including mixing titanium alkoxide with amine, adding water thereto, and reacting the mixture (Patent Literature 10) is known.

### CITATION LIST

### PATENT LITERATURE

Patent Literature 1: Japanese Unexamined Patent Publication No. 2001-322815
Patent Literature 2: Japanese Unexamined Patent Publication No. H11-158691
Patent Literature 3: Japanese Unexamined Patent Publication No. 2009-0132762
Patent Literature 4: Japanese Unexamined Patent Publication No. 2013-091594
Patent Literature 5: Japanese Unexamined Patent Publication No. 2012-062239
Patent Literature 6: Japanese Unexamined Patent Publication No. S55-113623
Patent Literature 7: Japanese Unexamined Patent Publication No. S62-21799
Patent Literature 8: Japanese Unexamined Patent Publication No. H02-164800
Patent Literature 9: Japanese Unexamined Patent Publication No. H06-275263
Patent Literature 10: Japanese Patent No. 3502904

### SUMMARY OF THE INVENTION

### PROBLEM TO BE SOLVED BY THE INVENTION

As described above, various methods are known for preparing titanate compounds composed of alkali metals or alkaline earth metals. However, many of the methods either require a process under high temperature or high pressure conditions, or a laborious process, to obtain the finished product. Therefore, it cannot be said that they are preferred methods for being adopted as industrial production methods.

Accordingly, an object of the present invention is to provide a novel titanate aqueous solution capable of easily preparing titanate compounds composed of alkali metals or alkaline earth metals, and a method for producing the same.

### MEANS FOR SOLVING PROBLEM

The present invention proposes a titanate aqueous solution containing titanate ions and quaternary ammonium cations in water, wherein 30 g of the titanate aqueous solution (25°C) adjusted to a concentration containing 9% by mass of titanium in terms of TiO₂ is added with 30 mL of a sodium hydroxide aqueous solution (25°C) having a concentration of 2.2% by mass while stirring to form a precipitate of composed of Na₂Ti₃O₇ hydrate.

The present invention also proposes a titanate aqueous solution containing titanate ions and quaternary ammonium cations in water, wherein the titanate aqueous solution has a transmittance at a wavelength of 360 nm being 50% or less.

The present invention further proposes a method for producing a titanate aqueous solution including: adding a titanium salt solution to an amine aqueous solution to obtain a neutralization reaction liquid (referred to as "neutralization step"); washing a titanium-containing precipitate formed in the neutralization reaction liquid (referred to as a "washing step"); and mixing the washed titanium-containing precipitate with a quaternary ammonium salt and water to prepare a titanate aqueous solution (referred to as "dissolving step").

### EFFECT OF THE INVENTION

The titanate aqueous solution proposed by the present invention can be effectively used in various industrial applications, such as coating the surfaces of various parts to form surface layers having various functions, and use as additives for catalysts. In addition, the titanate aqueous solution proposed by the present invention has high reactivity with hydroxides composed of alkali metals or alkaline earth metals, and thus when mixing with, for example, hydroxides composed of alkali metals or alkaline earth metals under normal temperature and pressure, metal titanate compounds composed of alkali metals or alkaline earth metals can be easily synthesized. These metal titanate compounds, such as lithium titanate, sodium titanate, potassium titanate, barium titanate, and strontium titanate, can be effectively used in various industrial applications.

### BRIEF DESCRIPTION OF DRAWINGS

FIG. 1 is an X-ray diffraction pattern obtained by subjecting a powder, which is obtained by drying a reactant (precipitate) obtained by reacting a titanate aqueous solution (sample) obtained in Example 1 with a sodium hydroxide aqueous solution, to powder X-ray diffraction measurement.
FIG. 2 is an X-ray diffraction pattern obtained by subjecting a powder, which is obtained by drying a reactant (precipitate) obtained by reacting a titanium-containing liquid (sample) obtained in Comparative Example 2 with a sodium hydroxide aqueous solution, to powder X-ray diffraction measurement.
FIG. 3 is an X-ray diffraction pattern obtained by subjecting a powder, which is obtained by drying a reactant (precipitate) obtained by reacting a titanium-containing liquid (sample) obtained in Comparative Example 3 with a sodium hydroxide aqueous solution, to powder X-ray diffraction measurement.
FIG. 4 is an X-ray diffraction pattern obtained by subjecting a powder, which is obtained by drying a reactant (precipitate) obtained by reacting a titanium-containing liquid (sample) obtained in Comparative Example 4 with a sodium hydroxide aqueous solution, to powder X-ray diffraction measurement.
FIG. 5 is an X-ray diffraction pattern obtained by subjecting a powder, which is obtained by drying a reactant (precipitate) obtained by reacting a titanate aqueous solution (sample) obtained in Example 1 with a barium hydroxide aqueous solution, to powder X-ray diffraction measurement.
FIG. 6 is an X-ray diffraction pattern of a substance obtained by drying a reactant (precipitate) obtained by reacting a titanate aqueous solution (sample) obtained in Example 1 with a barium hydroxide aqueous solution, followed by calcining.
FIG. 7 is an X-ray diffraction pattern obtained by subjecting a powder, which is obtained by drying a titanium-containing liquid (sample) obtained in Comparative Example 1, to powder X-ray diffraction measurement.
FIG. 8 is an X-ray diffraction pattern obtained by subjecting a powder, which is obtained by drying a titanium-containing liquid (sample) obtained in Comparative Example 2, to powder X-ray diffraction measurement.
FIG. 9 is an X-ray diffraction pattern obtained by subjecting a powder, which is obtained by drying a titanium-containing liquid (sample) obtained in Comparative Example 3, to powder X-ray diffraction measurement.
FIG. 10 is an X-ray diffraction pattern obtained by subjecting a powder, which is obtained by drying a titanate aqueous solution (sample) obtained in Example 1, to powder X-ray diffraction measurement.
FIG. 11 is an X-ray diffraction pattern obtained by subjecting a powder, which is obtained by drying a titanate aqueous solution (sample) obtained in Example 2, to powder X-ray diffraction measurement.
FIG. 12 is an X-ray diffraction pattern obtained by subjecting a powder, which is obtained by drying a titanate aqueous solution (sample) obtained in Example 3, to powder X-ray diffraction measurement.
FIG. 13 is an X-ray diffraction pattern obtained by subjecting a powder, which is obtained by drying a titanate aqueous solution (sample) obtained in Example 4, to powder X-ray diffraction measurement.
FIG. 14 is an X-ray diffraction pattern obtained by subjecting a powder, which is obtained by drying a titanium-containing liquid (sample) obtained in Comparative Example 4, to powder X-ray diffraction measurement.

### MODE(S) FOR CARRYING OUT THE INVENTION

Next, the present invention will be described based on exemplary embodiments. However, the present invention is not limited to the embodiments described below.

### <Present Titanate Aqueous Solution>

The titanate aqueous solution according to an example of the embodiments of the present invention (referred to as "present titanate aqueous solution") is a titanate aqueous solution containing titanate ions and quaternary ammonium cations in water.

In the present invention, the term "titanate aqueous solution" means a dispersion containing titanium or titanate ions in water, in which the transmittance at a wavelength of 450 nm is 70% or more.

Whether the present titanate aqueous solution contains quaternary ammonium cations in water can be confirmed by mass spectrometry (MS) or the like. However, it is not limited to this confirmation method.

In the present titanate aqueous solution, the titanate ions can be presumed to be trititanate ions. Specifically, it can be presumed to be Ti₃O₇²⁻.

To obtain a trititanate structure such as (NR₄⁺)₂·Ti₃O₇²⁻, 2 mol or more of cations (NR₄⁺) are required for 3 mol of titanium. It is also confirmed that when the present titanate aqueous solution is reacted with sodium hydroxide as described later, a precipitate presumed to be Na₂Ti₃O₇ hydrate is formed. From these points, it can be presumed that titanium is present as titanate ions, particularly as trititanate ions, and more particularly as Ti₃O₇²⁻ in the present titanate aqueous solution.

In the present titanate aqueous solution, the quaternary ammonium cations are preferably cations represented by the following general formula of NR₁R₂R₃R₄⁺ (wherein R₁ to R₄ each independently represents a linear, branched-chain, or cyclic hydrocarbon group, alkoxy group, benzoyl group (-COCeHs), or hydroxy group; and all of R₁ to R₄ may be different, all thereof may be the same, or some thereof may be the same).

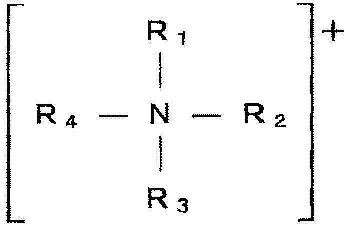

The present titanate aqueous solution preferably contains titanium in a ratio of 0.01% by mass to 15% by mass relative to 100% by mass of the aqueous solution, more preferably 0.1% by mass or more or 12% by mass or less, and even more preferably 0.2% by mass or more or 10% by mass or less, in terms of TiO₂.

The titanium or titanate in the present titanate aqueous solution is not necessarily present in the form of TiO₂. It may or may not be present in the form of TiO₂. The reason why the content of titanium is indicated in terms of TiO₂ is based on the practice of the industry.

The present titanate aqueous solution preferably contains quaternary ammonium cations in a molar ratio of 0.44 to 1.0 relative to titanium.

When the amount of the quaternary ammonium cations is large, the dispersibility or solubility of titanate in water can be enhanced. It can be presumed that the ion-bonding of quaternary ammonium cations with titanate enhances the solubility in water. From such a viewpoint, the present titanate aqueous solution preferably contains quaternary ammonium cations in a molar ratio of 0.44 or more, more preferably 0.45 or more, and even more preferably 0.46 or more, relative to titanium.

On the other hand, too much amount of quaternary ammonium cations may cause problems such as interfering with film formation and inhibiting catalytic action. Therefore, the present titanate aqueous solution preferably contains quaternary ammonium cations in a molar ratio of 1.0 or less, more preferably 0.80 or less, and even more preferably 0.65 or less, relative to titanium.

The present titanate aqueous solution is characterized in that, in an X-ray diffraction pattern obtained by subjecting a powder obtained by drying the present titanate aqueous solution to X-ray diffraction measurement using CuKα rays, peaks are present at respective positions of at least 2θ = 6.5° ± 1.4°, 15.5° ± 3°, and 26.0° ± 3° (referred to as "peak 1, 2, and 3", respectively). Among others, peaks are also preferably present at 2θ = 30.0° ± 3° and 48.5° ± 3° (referred to as "peak 4 and 5", respectively).

Here, the term "peak" refers to an inflection point having an intensity ratio of 4.0 or more relative to the intensity at 2θ = 85°.

Among others, the present titanate aqueous solution preferably has an intensity ratio (peak 2/peak M) of an intensity (peak 2 intensity) of the maximum peak at 2θ = 15.5° ± 3° to an intensity (peak M intensity) of the maximum peak at 2θ = 26.0° ± 3° being 0.6 to 2.0, more preferably 0.7 or more or 1.6 or less, even more preferably 0.75 or more or 1.4 or less, and still more preferably 0.8 or more or 1.3 or less, in an X-ray diffraction pattern obtained by subjecting a powder obtained by drying the present titanate aqueous solution to X-ray diffraction measurement using CuKα rays.

When the present titanate aqueous solution has such characteristics, it can be considered that the reactivity with hydroxides of alkali metals or alkaline earth metals can be increased.

In the present invention, the term "intensity of the maximum peak" means an intensity of the peak having the highest intensity among the peaks present in a predetermined diffraction angle range.

Furthermore, the present titanate aqueous solution preferably has an intensity ratio (peak 1/peak M) of an intensity (peak 1 intensity) of the maximum peak at 2θ = 6.5° ± 1.4° to an intensity (peak M intensity) of the maximum peak at 2θ = 26.0° ± 3° being 3.0 or more, in an X-ray diffraction pattern obtained by subjecting a powder obtained by drying the present titanate aqueous solution to X-ray diffraction measurement using CuKα rays.

When the intensity ratio (peak 1/peak M) of the intensity (peak 1 intensity) of the maximum peak at 2θ = 6.5° ± 1.4° to the intensity (peak M intensity) of the maximum peak at 2θ = 26.0° ± 3° is high, the dispersibility of the present titanate aqueous solution is enhanced and the transmittance of the present titanate aqueous solution is increased. In the X-ray diffraction pattern, the high intensity of the peak appearing at an angle lower than 2θ = 15° has been confirmed as one of the characteristics of a polyoxometalate structure. Therefore, it can be presumed that the higher the intensity ratio (peak 1/peak M), the higher the ratio of the polyoxometalate structure, the higher the dispersibility of the present titanate aqueous solution, and the higher the transmittance.

From such a viewpoint, the intensity ratio (peak 1/peak M) in the present titanate aqueous solution is preferably 3.0 or more, more preferably 3.1 or more, and even more preferably 3.2 or more. The upper limit thereof is expected to be approximately 15.0.

The above "2θ = 6.5° ± 1.4°" is preferably 2θ = 6.5° ± 1.0°.

The above "15.5° ± 3°" is preferably 15.5° ± 2.5°, more preferably 15.5° ± 2°, even more preferably 15.5° ± 1.5°, and still more preferably 15.5° ± 1°.

The above "26.0° ± 3°" is preferably 26.0° ± 2.5°, more preferably 26.0° ± 2°, even more preferably 26.0° ± 1.5°, and still more preferably 26.0° ± 1°.

The above "30.0° ± 3°" is preferably 30.0° ± 2.5°, more preferably 30.0° ± 2°, even more preferably 30.0° ± 1.5°, and still more preferably 30.0° ± 1°.

The above "48.5° ± 3°" is preferably 48.5° ± 2.5°, more preferably 48.5° ± 2°, even more preferably 48.5° ± 1.5°, and still more preferably 48.5° ± 1°.

### (Composition)

The present titanate aqueous solution may have a composition containing no components other than those derived from titanium or titanate and those derived from quaternary ammonium cations in water.

In this case, examples of the components derived from titanium or titanate include hydrates of titanium or titanate, or ions thereof.

Examples of the components derived from quaternary ammonium cations include quaternary ammonium ions, quaternary ammonium salts, and compounds composed of quaternary ammonium ions and titanium or titanate.

The present titanate aqueous solution may contain components other than those derived from titanium or titanate and those derived from quaternary ammonium cations (referred to as "other components") to the extent that they do not interfere with the effect.

Examples of the other components include Nb, Si, and Al. However, it is not limited to these.

The content of the other components in the present titanate aqueous solution is preferably less than 5% by mass, more preferably less than 4% by mass, and even more preferably less than 3% by mass.

It is also assumed that the present titanate aqueous solution contains unavoidable impurities, not intentionally. In this case, the content of unavoidable impurities is preferably less than 0.01% by mass.

In addition, the present titanate aqueous solution preferably contains no hardly-volatile organic components. When the present titanate aqueous solution contains no hardly-volatile organic components, it can be dried at a relatively low temperature (140°C or lower) to form a film, and it can also be effectively used for various applications such as catalyst raw materials since it contains no impurities.

Examples of the "hardly-volatile organic components" include triethanolamine, alkanolamine, oxycarboxylic acid, other chelating agents, ethylenediamine tetraacetate, citrate, nitrilotriacetate, cyclohexanediamine tetraacetate, other complexing agents, glycol, EDTA, amine, amine compounds, oxalic acid, butyric acid, organic metal compounds, halides, aniline, and nitrobenzene; and these are organic substances having a volatilization temperature of 150°C or higher.

Whether the present titanate aqueous solution contains "no hardly-volatile organic components" can be confirmed from the production method. If the production method is unknown, it can be confirmed by analyzing the presence or absence of the hardly-volatile organic components using, for example, gas chromatography, nuclear magnetic resonance (NMR), or GC-MS.

In this case, the present titanate aqueous solution contains "no hardly-volatile organic components" means that the content of organic substances having a volatilization temperature of 150°C or higher is less than 1%.

### (Transmittance)

The present titanate aqueous solution preferably has a transmittance at a wavelength of 450 nm being 70% or more, more preferably 80% or more, even more preferably 90% or more, and still more preferably 100%.

In addition, the present titanate aqueous solution preferably has a transmittance at a wavelength of 360 nm being 50% or less, more preferably 45% or less, even more preferably 40% or less, and still more preferably 35% or less.

### (Reactivity)

The present titanate aqueous solution has high reactivity with hydroxides composed of alkali metals or alkaline earth metals, and can be thus mixed and reacted with aqueous solutions composed of alkali metals or alkaline earth metal salts under normal temperature and pressure to obtain titanate compounds composed of alkali metals or alkaline earth metals.

Therefore, when the present titanate aqueous solution is mixed with hydroxides composed of, for example, lithium, sodium, potassium, barium, and strontium under normal temperature and pressure, titanate compounds composed of alkali metals or alkaline earth metals, such as lithium titanate, sodium titanate, potassium titanate, barium titanate, and strontium titanate, can be obtained.

Generally, in order to obtain titanates composed of alkali metals or alkaline earth metals such as Na₂Ti₃O₇ hydrate and Ba₂Ti₃O₇ hydrate, it is necessary to mix with hydroxides composed of alkali metals or alkaline earth metals and react the mixture under high temperature and pressure conditions using an autoclave or the like. Therefore, it is known to be difficult to produce titanates easily since the materials need to be heated to at least 80°C or higher for reaction.

On the other hand, the present titanate aqueous solution has high reactivity with hydroxides composed of alkali metals or alkaline earth metals, and it is thus possible to obtain titanate compounds by simply mixing and reacting with hydroxides composed of alkali metals or alkaline earth metals under normal temperature and pressure.

Other than the present titanate aqueous solution, there are no known titanium aqueous solutions capable of synthesizing Na₂Ti₃O₇ hydrate (sodium trititanate) under normal temperature and pressure.

For example, 30 g of the present titanate aqueous solution (25°C) adjusted to a concentration containing 9% by mass of titanium in terms of TiO₂ is added with 30 mL of a sodium hydroxide aqueous solution (25°C) having a concentration of 2.2% by mass while stirring to immediately form a precipitate, the stirring is continued for 30 minutes or less, if necessary, to age the precipitate, and the formed precipitate is then dried, thereby obtaining a powder composed of Na₂Ti₃O₇ hydrate (sodium trititanate).

From the above, it can be defined that the present titanate aqueous solution can be reacted with a sodium hydroxide aqueous solution as described above to form a precipitate composed of Na₂Ti₃O₇ hydrate.

When the sodium trititanate is subjected to X-ray diffraction measurement using Cu-Ka rays in a range of 2θ = 5° to 90°, one or more peaks of the sodium trititanate compound is observed.

The compound preferably has a main peak observed at 2θ = 10° or less in the X-ray diffraction measurement. The peak detected at 10° or less means that the compound has a crystal orientation of (001), and the peak is derived from Na₂Ti₃O₇ hydrate.

When a main peak is detected at 2θ = 10° or less, it is preferable that peaks are further detected in at least one of a range of 2θ = 27° or more and 29° or less, and a range of 47° or more and 49° or less.

Here, the peaks detected at 2θ = 27° or more and 29° or less, and 2θ = 47° or more and 49° or less preferably have an intensity of 10% or more and 70% or less, relative to the intensity of the main peak.

For the trititanate, "Alkali metal intercalated titanate nanotubes: A vibrational spectroscopy study", Bartolomeu C. Vianaet al., Vibrational Spectroscopy 55 (2011) 183-187 (reference document) may be referred.

In addition, 30 g of the present titanate aqueous solution (25°C) adjusted to a concentration containing 9% by mass of titanium in terms of TiO₂ is added with 30 mL of a barium hydroxide aqueous solution (25°C) having a concentration of 2.2% by mass while stirring to immediately form a precipitate, the stirring is continued for 30 minutes or less, if necessary, to age the precipitate, and the formed precipitate is then dried, thereby obtaining a powder composed of BaTi₃O₇ hydrate.

From the above, it can be defined that the present titanate aqueous solution can be reacted with a barium hydroxide aqueous solution as described above to form a precipitate composed of BaTi₃O₇ hydrate.

Whether the precipitate thus formed is a precipitate composed of Na₂Ti₃O₇ hydrate or BaTi₃O₇ hydrate can be identified by, for example, the following X-ray diffraction measurement (XRD). However, it is not limited to this method.

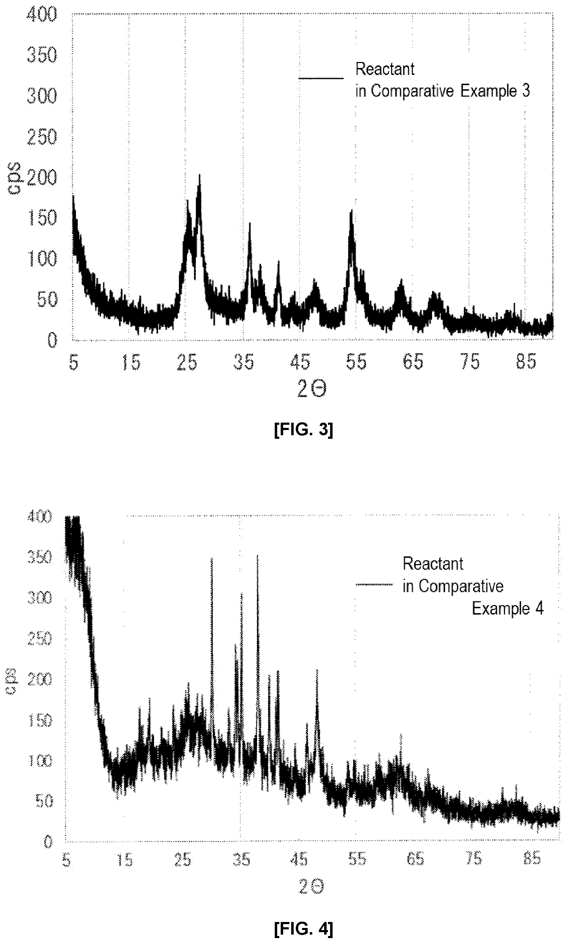

Specifically, the precipitate thus formed is measured by X-ray diffraction measurement under the following conditions, and the XRD pattern is compared with that described in FIG. 3 (b) of the above-mentioned reference document, thereby identifying as Na₂Ti₃O₇ hydrate.

Whether it is Na₂Ti₃O₇ hydrate can also be presumed from the fact that the XRD pattern is completely different from that of Na₂Ti₃O₇ anhydride (ICDD card No. 31-1329).

On the other hand, the BaTi₃O₇ hydrate can be identified by calcining the formed precipitate, for example, at 1,000°C for 2 hours, and identifying whether the calcined product is composed of BaTi₂O₅ and BaTi₄O₉.

### <Present Production Method>

Next, a preferred method for producing the present titanate aqueous solution (referred to as "present production method") will be described.

Examples of the present production method include a method for producing a titanate aqueous solution including: mixing a titanium salt solution with an amine aqueous solution to obtain a neutralization reaction liquid (referred to as "neutralization step"); washing a titanium-containing precipitate formed in the neutralization reaction liquid (referred to as "washing step"); and mixing the washed titanium-containing precipitate with a quaternary ammonium salt and water to prepare a titanate aqueous solution (referred to as "dissolving step"). However, the method for producing the present titanate aqueous solution is not limited to this production method.

Other steps or treatments can be appropriately added in the present production method as long as the above-mentioned steps are included.

For ease of explanation, the present production method will be described for each step below. However, each step can be a series of treatments in terms of an apparatus and time, or can be different treatment steps with different apparatuses and times.

### (Titanium Salt Solution)

The titanium salt solution may be a solution in which titanium is dissolved. Examples thereof include a titanyl sulfate aqueous solution, a titanium chloride aqueous solution, and a titanium fluoride aqueous solution.

The titanium chloride aqueous solution can be prepared by dissolving titanium chloride (TiCl₅) into a small amount of methanol and then adding water thereto.

The titanyl sulfate aqueous solution can be prepared by dissolving titanyl sulfate into hot water.

The titanyl sulfate aqueous solution is preferably prepared so as to contain 8% by mass to 15% by mass of titanium in terms of TiO₂.

### (Neutralization Step)

In the present production method, a titanium salt solution may be mixed and reacted with an amine aqueous solution to obtain a neutralization reaction liquid.

In the neutralization step, it is preferable to perform reverse neutralization, in which a titanium salt solution such as a titanyl sulfate aqueous solution is added to an amine aqueous solution for reaction.

With the reverse neutralization as described above, it is presumed that the structure of titanium or titanate becomes a structure that is more soluble in water.

Preferred examples of the amine in the amine aqueous solution used in the neutralization step include alkylamines.

As the alkylamines, those having 1 to 3 alkyl groups can be preferably used. When the number of alkyl groups is 2 to 3, all of those alkyl groups may be the same or different. As the alkyl groups of the alkylamines, alkyl groups having 1 to 6 carbon atoms, preferably 4 or less, more preferably 3 or less, and even more preferably 2 or less, are preferred from the viewpoint of solubility.

Specific examples of the alkylamines include methylamine, dimethylamine, trimethylamine, ethylamine, methylethylamine, diethylamine, triethylamine, methyldiethylamine, dimethylethylamine, n-propylamine, di-n-propylamine, tri-n-propylamine, iso-propylamine, di-iso-propylamine, tri-iso-propylamine, n-butylamine, di n-butylamine, tri-n-butylamine, iso-butylamine, di-iso-butylamine, tri-iso-butylamine, tert-butylamine, n-pentaamine, and n-hexaamine.

Among them, from the viewpoint of solubility, methylamine, dimethylamine, trimethylamine, ethylamine, methylethylamine, diethylamine, triethylamine, methyldiethylamine, and dimethylethylamine are preferred; and methylamine, dimethylamine, and trimethylamine are more preferred.

In the neutralization step, from the viewpoint of enhancing dispersibility, it is preferable to add the titanium salt solution to an amine aqueous solution containing amine in a molar ratio equivalent to or more than sulfuric acid contained in the titanium salt solution, that is, in a molar ratio of 1 or more, more preferably 1.2 or more, and even more preferably 1.4 or more, relative to sulfuric acid contained in the titanium salt solution.

In addition, from the viewpoint of reducing the amount of waste solution, it is preferable to add the titanium salt solution to an amine aqueous solution containing amine in a molar ratio of 2 or less, more preferably 1.8 or less, and even more preferably 1.6 or less, relative to sulfuric acid contained in the titanium salt solution.

In the neutralization step, it is preferable to perform neutralization reaction within 1 minute when adding the titanium salt solution such as a titanyl sulfate aqueous solution to an amine aqueous solution. In other words, instead of gradually adding the titanium salt solution over a period of time, it is preferable to add the titanium salt solution all at once, for example, within 1 minute for neutralization reaction.

In this case, the addition time of the titanium salt solution is preferably 1 minute or less, more preferably 30 seconds or less, and even more preferably 10 seconds or less.

### (Washing Step)

In the neutralization reaction liquid obtained in the neutralization step, especially in the titanium-containing precipitate, impurities, which are unnecessary components other than hydrates of titanium or titanate or ions thereof and amine, such as sulfate compounds including ammonium sulfate, are present in water. Therefore, it is preferable to remove the unnecessary components by washing.

The washing method, for example, the method for removing sulfate compounds, is arbitrary. For example, filtration methods using membranes, such as reverse osmosis filtration using ammonia water or pure water, ultrafiltration, and microfiltration; centrifugation; and other known methods can be adopted.

The washing step may be performed at room temperature, and each temperature adjustment is not particularly required.

### (Dissolving Step)

Next, the titanium-containing precipitate obtained by washing in the washing step, for example, the titanium-containing precipitate obtained by removing sulfuric acid, can be added with a dispersion medium such as water, and a quaternary ammonium salt, and then stirred if necessary to promote the reaction, thereby producing the present titanate aqueous solution.

Examples of the quaternary ammonium salt to be added include tetramethylammonium hydroxide, tetraethylammonium hydroxide, tetrapropylammonium hydroxide, tetrabutylammonium hydroxide, methyltripropylammonium hydroxide, methyltributylammonium hydroxide, tetrapentylammonium hydroxide, tetrahexylammonium hydroxide, ethyltrimethylammonium hydroxide, dimethyldiethylammonium hydroxide, benzyltrimethylammonium hydroxide, hexadecyltrimethylammonium hydroxide, and (2-hydroxyethyl)trimethylammonium hydroxide.

It is confirmed that when a primary to tertiary amine or salt thereof is added in place of the quaternary ammonium salt, the precipitate cannot be made into a form of aqueous solution.

As for the amount of the quaternary ammonium salt to be added, as described above, when the amount of quaternary ammonium is large, the solubility of titanium or titanate in water can be enhanced. Therefore, in the dissolving step, it is preferable to mix a quaternary ammonium salt containing quaternary ammonium in 0.44 mol or more per 1 mol of titanium contained in the washed titanium-containing precipitate.

On the other hand, when the amount of quaternary ammonium is too large, problems such as interfering with film formation and inhibiting catalytic action may occur. From such a viewpoint, in the dissolving step, it is preferable to mix a quaternary ammonium salt containing quaternary ammonium in 1.0 mol or less per 1 mol of titanium contained in the washed titanium-containing precipitate.

Each step in the present production method may be performed at room temperature, and each temperature adjustment is not particularly required.

### <Applications>

The present titanate aqueous solution can be dried at a relatively low temperature (140°C or lower) to form a film. Therefore, it can be effectively used as, for example, various coating liquids. It can also be used for various other applications, such as catalyst raw materials.

As described above, the present titanate aqueous solution has high reactivity with hydroxides composed of alkali metals or alkaline earth metals. Therefore, when mixing with, for example, hydroxides composed of alkali metals or alkaline earth metals under normal temperature and pressure, metal titanate compounds composed of alkali metals or alkaline earth metals can be easily synthesized. These metal titanate compounds, such as lithium titanate, sodium titanate, potassium titanate, barium titanate, and strontium titanate, can be effectively used in various industrial applications.

### <Explanation of Terms>

The expression "X to Y" (X and Y are arbitrary numbers) in the present specification also includes the intention of "preferably more than X" or "preferably less than Y" as well as the intention of "X or more and Y or less" unless otherwise stated.

In addition, the expression "X or more" (X is an arbitrary number) or "Y or less" (Y is an arbitrary number) also includes the intention of "preferably more than X" or "preferably less than Y".

### EXAMPLES

The present invention will be further described based on the following Examples. However, the following Examples are not intended to limit the present invention.

### (Example 1)

Titanyl sulfate (TiO₂ concentration of 33.3% by mass, sulfuric acid concentration of 51.1% by mass, manufactured by Tayca Corp.) in an amount of 33.3 g was added to 66.7 g of ion-exchanged water, and left to stand at 90°C or higher for 1 hour to be dissolved, thereby obtaining a titanyl sulfate aqueous solution (titanium concentration of 11% by mass (in terms of TiO₂), sulfuric acid concentration of 17% by mass, and pH value of 1 or less).

The titanyl sulfate aqueous solution in an amount of 100 g was added to 100 g of a 50% dimethylamine (6.4 mol of amine per 1 mol of sulfuric acid in the titanyl sulfate aqueous solution) over a period of less than 1 minute. The mixture was then stirred for 15 minutes to obtain a neutralization reaction liquid (pH 12). The neutralization reaction liquid was a titanium-containing substance slurry, in other words, a titanium-containing precipitate slurry.

Then, the neutralization reaction liquid was decanted using a centrifuge and washed until the amount of sulfuric acid in the supernatant was 100 mg/L or less to obtain a titanium-containing precipitate from which sulfuric acid was removed. Ammonia water was used for the washing liquid in this process.

A part of the titanium-containing precipitate was calcined at 1,000°C for 4 hours to form TiO₂, and the concentration of TiO₂ contained in the titanium-containing precipitate was calculated from the mass. The TiO₂ concentration was 11.0% by mass.

Next, 45 g of the titanium-containing precipitate was mixed with 5 g (0.443 mol per 1 mol of Ti in the titanium-containing precipitate) of tetramethylammonium hydroxide pentahydrate (TMAH concentration of 50% by mass), and the mixture was shaken using a paint shaker for 24 hours to obtain a titanate aqueous solution (sample).

As shown in Table 1, in 50 g of the titanate aqueous solution (sample), the content of TiO₂ was 4.95 g (0.062 mol), that is, 9.9% by mass, and the content of the quaternary ammonium cations was 2.5 g (0.027 mol), that is, 5.0% by mass.

### (Example 2)

A titanate aqueous solution (sample) was obtained in the same manner as in Example 1, except that 44 g of the titanium-containing precipitate was mixed with 6 g (0.542 mol per 1 mol of Ti in the titanium-containing precipitate) of tetramethylammonium hydroxide pentahydrate (TMAH concentration of 50% by mass) in Example 1.

As shown in Table 1, in 50 g of the titanate aqueous solution (sample), the content of TiO₂ was 4.85 g (0.061 mol), that is, 9.7% by mass, and the content of the quaternary ammonium cations was 3.0 g (0.033 mol), that is, 6.0% by mass.

### (Example 3)

A titanate aqueous solution (sample) was obtained in the same manner as in Example 1, except that 43 g of the titanium-containing precipitate was mixed with 7 g (0.613 mol per 1 mol of Ti in the titanium-containing precipitate) of tetramethylammonium hydroxide pentahydrate (TMAH concentration of 50% by mass) in Example 1.

As shown in Table 1, in 50 g of the titanate aqueous solution (sample), the content of TiO₂ was 4.75 g (0.063 mol), that is, 9.5% by mass, and the content of the quaternary ammonium cations was 3.5 g (0.038 mol), that is, 7.0% by mass.

### (Example 4)

A titanate aqueous solution (sample) was obtained in the same manner as in Example 1, except that 22.7 g of the titanium-containing precipitate was mixed with 4.8 g (0.521 mol per 1 mol of Ti in the titanium-containing precipitate) of tetraethylammonium hydroxide (TEAH concentration of 50% by mass) and 22.5 g of ion-exchanged water in Example 1.

As shown in Table 1, in 50 g of the titanate aqueous solution (sample), the content of TiO₂ was 2.50 g (0.031 mol), that is, 5.0% by mass, and the content of the quaternary ammonium cations was 2.4 g (0.016 mol), that is, 4.8% by mass.

### (Comparative Example 1)

In Example 1, 44 g of the titanium-containing precipitate was mixed with 6 g (1.096 mol per 1 mol of Ti in the titanium-containing precipitate) of 50% dimethylamine. However, the mixture was immediately gelled to lose fluidity, thereby obtaining no titanate aqueous solution.

As shown in Table 1, in 50 g of the gel-shaped substance (also referred to as "titanium-containing liquid (sample)"), the content of TiO₂ was 4.85 g (0.061 mol), that is, 9.7% by mass, and the content of amine was 3.0 g (0.067 mol), that is, 6.0% by mass.

### (Comparative Example 2)

Lactic acid in an amount of 90 g was added with 30 g of ion-exchanged water to be dissolved, and the lactic acid aqueous solution was added with 142 g of tetraisopropyl orthotitanate while stirring at room temperature, followed by heating to 60°C. Next, 34.3 g of 25% ammonia water was added thereto, and the mixture was further reacted at 60°C to obtain a pale yellow titanium lactate ammonium aqueous solution. The titanium lactate ammonium aqueous solution was diluted with 204.8 g of ion-exchanged water to obtain an aqueous solution having a TiO₂ concentration of 10.0% by mass (also referred to as "titanium-containing liquid (sample)").

As shown in Table 1, in 50 g of the titanium-containing liquid (sample), the content of TiO₂ was 5.00 g (0.063 mol), that is, 10.0% by mass, the content of lactic acid was 12.8 g (0.142 mol), that is, 25.6% by mass, and the content of ammonia was 2.85 g (0.168 mol), that is, 5.7% by mass.

### (Comparative Example 3)

A 3.2% sodium hydroxide aqueous solution in an amount of 10,361 g was added with 600 g of a titanium oxychloride aqueous solution (TiO₂: 27.5%, Cl: 33.0%) over a period of 30 minutes while stirring. The pH value of the resulting hydrated titanium oxide gel was 13. This was decanted using a centrifuge and washed until the filtrate EC was 5.0 mS/cm or less to obtain a titanium-containing precipitate.
Ion-exchanged water was used for washing.

The titanium-containing precipitate was added with ion-exchanged water to adjust the titanium concentration to 4.5% by mass.

The titanium-containing slurry in an amount of 800 g was added with 44 g of pure water and 56.3 g of 35% hydrochloric acid (pH 0.8). This solution was heated to 60°C, and after 30 minutes, a 18% ammonia aqueous solution was added thereto until the pH value was 8. This was then heated at 95°C for 2 hours. This was washed by ultrafiltration until the filtrate EC was 35 µS/cm to obtain a titanium oxide gel having anatase-type and rutile-type crystal structures (TiO₂: 10.5%, pH 8.1, EC: 0.56 mS/cm, and specific surface area: 275 m²/g). The titanium oxide gel in an amount of 300 g was added with 14.3 g of a 25% tetramethylammonium hydroxide aqueous solution (25% TMAH), and heated at 90°C for 3 hours to obtain an alkaline titanium oxide sol (also referred to as "titanium-containing liquid (sample)").

As shown in Table 1, in 50 g of the titanium-containing liquid (sample), the content of TiO₂ was 5.00 g (0.063 mol), that is, 10.0% by mass, and the content of the quaternary ammonium cations was 0.6 g (0.007 mol), that is, 1.2% by mass. However, since the titanium-containing liquid (sample) is a sol, it is clear that titanium is not present as titanate ions.

### (Comparative Example 4)

TIP (titanium tetraisopropoxide) in an amount of 13.5 g (0.052 mol in terms of TiO₂) and 15% TMAH (tetramethylammonium hydroxide) in an amount of 36.5 g (0.052 mol in terms of TMAH) were mixed and stirred for 30 minutes to be reacted, thereby obtaining a colorless and transparent Ti-containing aqueous solution (TiO₂ concentration of 8.3% by mass).

### <Reactivity Test with NaOH>

The titanate aqueous solution (sample) obtained in each of Examples 1 to 4 or the titanium-containing liquid (sample) obtained in each of Comparative Examples 2 to 3 (25°C) in an amount of 30 g was weighed, and 30 mL of a 2.2% by mass sodium hydroxide aqueous solution (25°C) was added thereto over a period of 1 minute while stirring using a magnetic stirrer (stirring speed: 150 rpm). After the addition, the reaction immediately proceeded to obtain a reactant (precipitate), which was stirred for another 15 minutes to be aged. The precipitate was subjected to Nutsche filtration using 5C filter paper, washed with pure water, and then dried by still-standing for 5 hours in an atmosphere of 90°C and vacuum (0.08 MPa or less) using a reduced pressure drying furnace. The resulting dried product was pulverized in an agate mortar, and the resulting powder was subjected to X-ray diffraction measurement.

Here, the X-ray diffraction measurement conditions and the X-ray diffraction conditions were the same as those of the following <XRD measurement>. However, the peak smoothing with b-spline was not performed in the analysis of the reactant.

The titanium-containing liquid (sample) obtained in Comparative Example 1 was not performed for the above reactivity test since it was gelled and had no fluidity.

The aqueous solution obtained in Comparative Example 4 was also performed for the same test. However, it solidified into a gel when adding a sodium hydroxide aqueous solution, and it was thus dried as it was by still-standing for 5 hours in an atmosphere of 90°C and a vacuum (0.08 MPa or less) using a reduced pressure drying furnace. The resulting dried product was pulverized in an agate mortar, and the resulting powder was subjected to X-ray diffraction measurement.

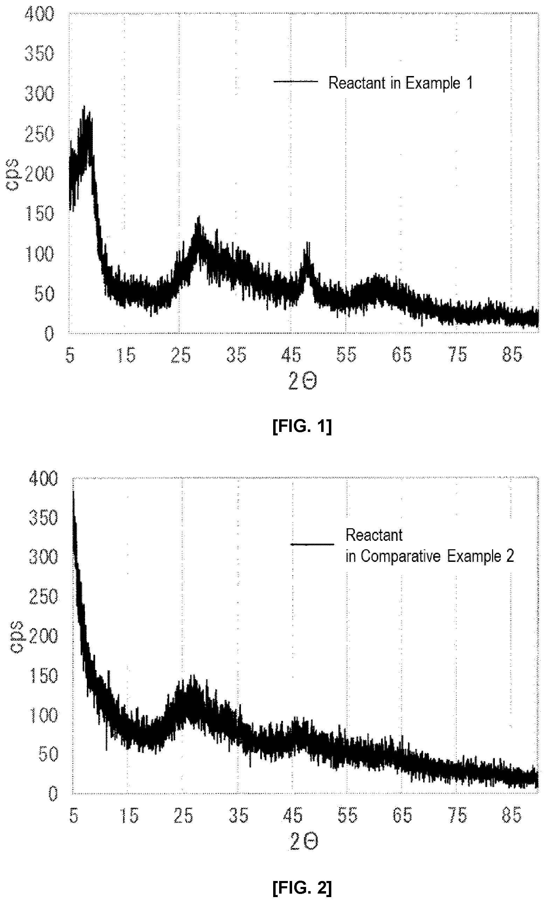

FIGS.1 to 4 each show an X-ray diffraction pattern of the powder obtained by reacting each of the titanate aqueous solution (sample) obtained in Example 1 (FIG. 1), the titanium-containing liquid (sample) obtained in Comparative Examples 2 (FIG. 2), the titanium-containing liquid (sample) obtained in Comparative Example 3 (FIG. 3), and the titanium-containing liquid (sample) obtained in Comparative Example 4 (FIG. 4), with a sodium hydroxide aqueous solution.

The powder obtained by reacting the titanate aqueous solution (sample) obtained in each of Examples 1 to 4 with a sodium hydroxide aqueous solution was identified to be composed of Na₂Ti₃O₇ hydrate, from the X-ray diffraction measurement results.

The powder obtained by reacting the titanium-containing liquid (sample) obtained in Comparative Example 2 with a sodium hydroxide aqueous solution was amorphous, and no titanium oxide was obtained, from the X-ray diffraction measurement results.

The powder obtained by reacting the titanium-containing liquid (sample) obtained in Comparative Example 3 with a sodium hydroxide aqueous solution was titanium oxide having normal anatase-type and rutile-type structures, and no titanate compound was obtained.

The powder obtained by reacting the titanium-containing liquid (sample) obtained in Comparative Example 4 with a sodium hydroxide aqueous solution was at least not Na₂Ti₃O₇ hydrate.

The reactivity with NaOH was evaluated according to the following criteria. The results are shown in Table 1.
○ (good): Those having a diffraction pattern presumed to be Na₂Ti₃O₇ hydrate
× (poor): Those having a diffraction pattern presumed not to be Na₂Ti₃O₇ hydrate

The reactivity test with NaOH was not performed in Comparative Example 1 as described above. The result is thus described with "-" in Table 1.

### <Reactivity Test with BaOH>

The titanate aqueous solution (sample) obtained in Example 1 in an amount of 30 g was weighed, and 207 g of a 3.3% by mass barium hydroxide aqueous solution was added thereto over a period of 1 minute while stirring using a magnetic stirrer (stirring speed: 150 rpm). After the addition, the reaction immediately proceeded to obtain a reactant (precipitate), which was stirred for another 15 minutes to be aged. The precipitate was subjected to Nutsche filtration using 5C filter paper, washed with pure water, and then dried by still-standing for 5 hours in an atmosphere of 90°C and vacuum (0.08 MPa or less) using a reduced pressure drying furnace. The resulting dried product was pulverized in an agate mortar, and the resulting powder was subjected to X-ray diffraction measurement.

Here, the X-ray diffraction measurement conditions and the X-ray diffraction conditions were the same as those of the following <XRD measurement>. However, the peak smoothing with b-spline was not performed in the analysis of the reactant.

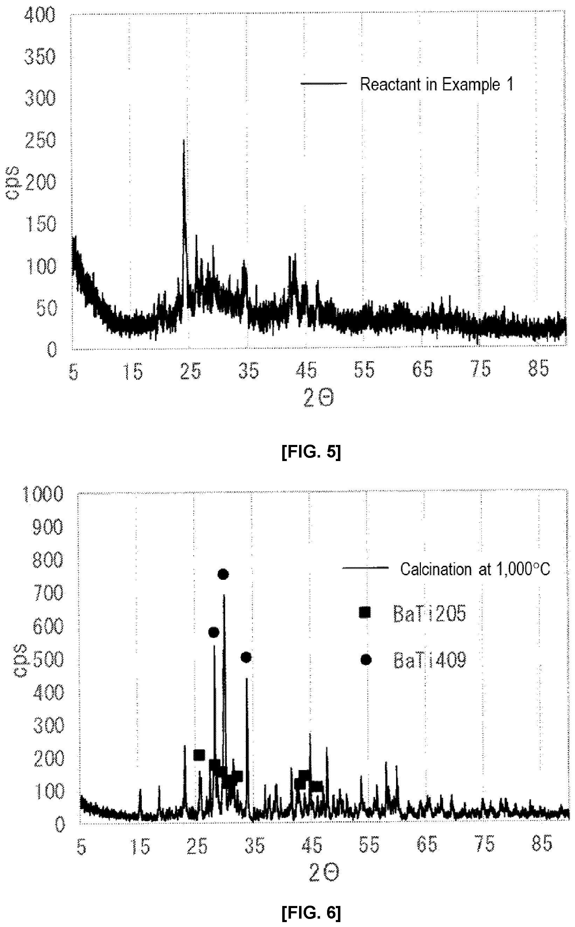

FIG. 5 shows an X-ray diffraction pattern of the powder obtained by reacting the titanate aqueous solution (sample) obtained in Example 1 with a barium hydroxide aqueous solution. This substance was not identified since it was unknown. Then, the substance was calcined at 1,000°C for 1 hour, and it was identified to be composed of BaTi₄O₉ and BaTi₂O₅. FIG. 6 shows an X-ray diffraction pattern of the substance after the calcination. For the identification, ICDD (PDF-2, 2021) card No.: 01-077-1565 and card No.: 00-034-0133 were used.

As a result, the powder obtained by reacting the titanate aqueous solution (sample) obtained in Example 1 with a barium hydroxide aqueous solution was presumed to be BaTi₃O₇ hydrate, that is, BaTi₃O₇·nH₂O.

### <Transmittance Measurement>

The transmittance of the titanate aqueous solution (sample) obtained in each of Examples 1 to 4 or the titanium-containing liquid (sample) obtained in each of Comparative Examples 1 to 3 was measured using a spectrophotometer.

### = Transmittance Measurement Conditions =

- Apparatus: UH4150-type spectrophotometer
- Measurement mode: Wavelength scanning
- Data mode: %T (transmission)
- Measurement wavelength range: 200 to 2,600 nm
- Scanning speed: 600 nm/min
- Sampling interval: 2 nm

The transmittances at wavelengths of 360 nm and 450 nm were calculated from the transmittance thus measured. The results are shown in Table 1.

The solubility was evaluated according to the following criteria. The results are shown in Table 1.
○ (good): Those having a transmittance at a wavelength of 450 nm of 70% or more
× (poor): Those having a transmittance at a wavelength of 450 nm of less than 70%

### <XRD Measurement>

The titanate aqueous solution (sample) obtained in each of Examples 1 to 4 or the titanium-containing liquid (sample) obtained in each of Comparative Examples 1 to 4 in an amount of 10 g was dried by still-standing at 110°C for 15 hours in the air using a drying furnace, and further dried by still-standing at 150°C for 2 hours in the air, thereby obtaining a titanate compound powder (sample).

Each titanate compound powder (sample) was subjected to powder X-ray diffraction measurement using CuKα rays to obtain an X-ray diffraction pattern.

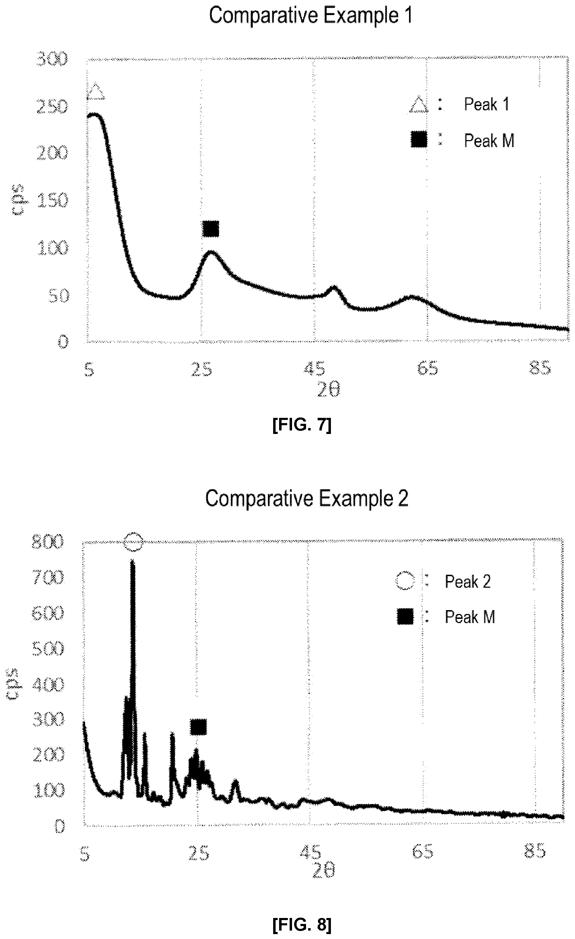

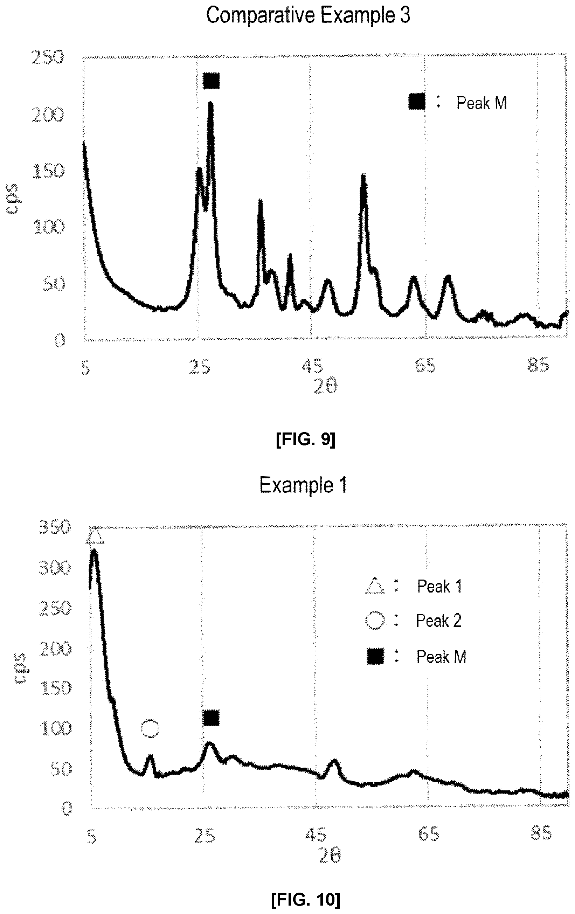

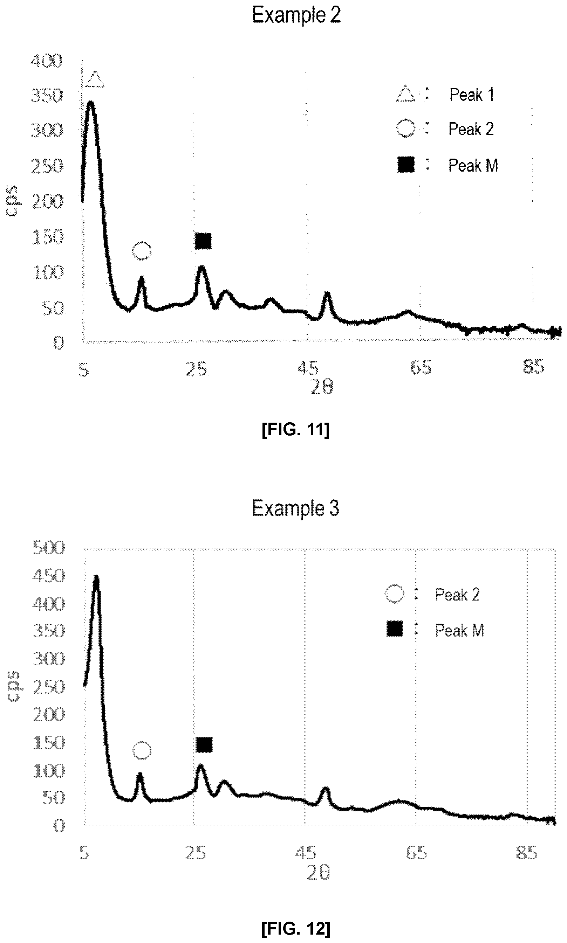

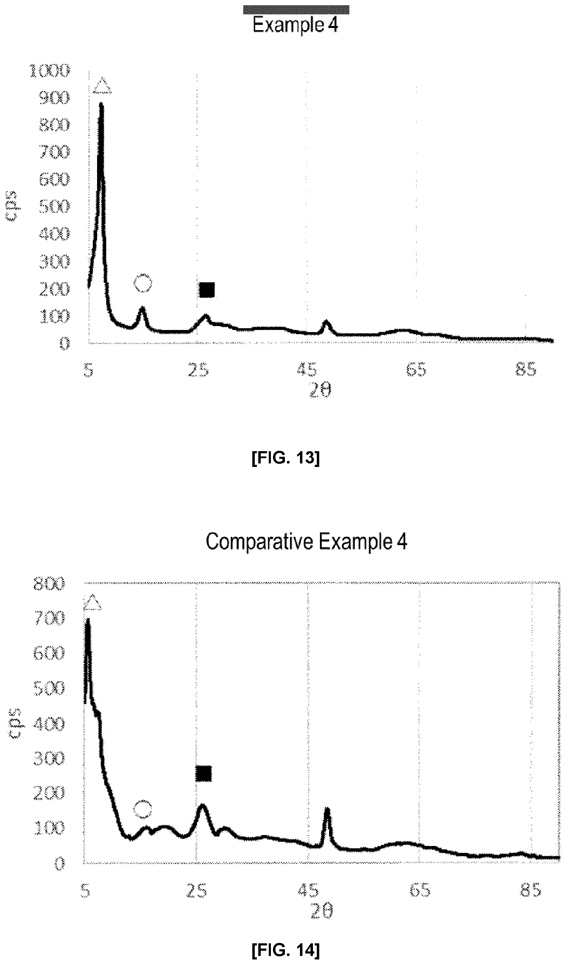

FIGS. 7 to 9 each show an X-ray diffraction pattern of the titanate compound powder (sample) obtained from the titanium-containing liquid (sample) obtained in each of Comparative Examples 1 to 3; FIGS. 10 to 13 each show an X-ray diffraction pattern of the titanate compound powder (sample) obtained from the titanate aqueous solution (sample) obtained in each of Examples 1 to 4; and FIG. 14 shows an X-ray diffraction pattern of the titanate compound powder (sample) obtained from the titanium-containing liquid (sample) obtained in Comparative Example 4.

### = X-Ray Diffraction Measurement Conditions =

- Apparatus: Mini Flex II (manufactured by Rigaku Corp.)
- Measurement range (2θ): 5° to 90°
- Sampling width: 0.02°
- Scanning speed: 2.0°/min
- X-ray: CuKα ray
- Voltage: 30 kV
- Current: 15 mA
- Divergence slit: 1.25°
- Scattering slit: 1.25°
- Light-receiving slit: 0.3 mm

### = X-Ray Diffraction Analysis Conditions =

- Data analysis software PDXL2, manufactured by Rigaku Corp., was used.
- Peaks were smoothed with a b-spline curve to clarify peak tops.

In the X-ray diffraction pattern obtained thus measured, peaks present at 2θ = 6.5° ± 1.4°, 15.5° ± 3°, 26.0° ± 3°, 30.0° ± 3°, and 48.5° ± 3° are referred to as peak 1, 2, 3, 4, and 5, respectively, and the diffraction angle and the peak intensity of each peak are shown in Table 2.

The diffraction angle and the peak intensity of the maximum peak (peak M) present at 2θ = 26.0° ± 3° are also shown in Table 2.

Furthermore, the ratio (peak 2/peak M) of the intensity of the peak 2 present at 2θ = 15.5° ± 3° to the intensity of the maximum peak (peak M) present at 2θ = 26.0° ± 3° is calculated and shown in Table 2.

In addition, the ratio (peak 1/peak M) of the intensity of the peak 1 present at 2θ = 6.5° ± 1.4° to the intensity of the maximum peak (peak M) present at 2θ = 26.0° ± 3° is calculated and shown in Table 2.

**[Table 1]**

| | | Comparative Examples | | | | Examples | | | |
|---|---|---|---|---|---|---|---|---|---|
| | | 1 | 2 | 3 | 4 | 1 | 2 | 3 | 4 |
| Liquid amount | g | 50 | 50 | 50 | 50 | 50 | 50 | 50 | 50 |
| TiO2 | % by mass | 9.7 | 10.0 | 10.0 | 8.3 | 9.9 | 9.7 | 9.5 | 5.0 |
| | g | 4.85 | 5.00 | 5.00 | 4.17 | 4.95 | 4.85 | 4.75 | 2.50 |
| | mol | 0.061 | 0.063 | 0.063 | 0.052 | 0.062 | 0.061 | 0.063 | 0.031 |
| TMAH | % by mass | | | 1.2 | 9.5 | 5.0 | 6.0 | 7.0 | |
| | g | | | 0.6 | 4.8 | 2.5 | 3.0 | 3.5 | |
| | mol | | | 0.007 | 0.052 | 0.027 | 0.033 | 0.038 | |
| Dimethylamine | % by mass | 6.0 | | | | | | | |
| | g | 3.0 | | | | | | | |
| | mol | 0.067 | | | | | | | |
| TEAH | % by mass | | | | | | | | 4.8 |
| | g | | | | | | | | 2.4 |
| | mol | | | | | | | | 0.016 |
| Lactic acid | % by mass | | 25.6 | | | | | | |
| | g | | 12.8 | | | | | | |
| | mol | | 0.142 | | | | | | |
| Ammonia | % | | 5.7 | | | | | | |
| | g | | 2.85 | | | | | | |
| | mol | | 0.168 | | | | | | |
| Amine/TiO2 (molar ratio) | | 1.10 | 0.00 | 0.11 | 1.00 | 0.44 | 0.54 | 0.61 | 0.52 |
| Liquid state | | gelled | aqueous solution | suspension | aqueous solution | aqueous solution | aqueous solution | aqueous solution | aqueous solution |
| Liquid color | | - | yellow | white | colorless | yellow | yellow | yellow | yellow |
| Light transmittance at 360 nm (%) | | 0 | 7 | 0.5 | 100 | 5 | 6 | 6 | 32 |
| Liqht transmittance at 450 nm (%) | | 0 | 100 | 0.7 | 100 | 100 | 100 | 100 | 100 |
| Solubility determination | | × | ○ | × | ○ | ○ | ○ | ○ | ○ |
| Reactivity with NaOH | | - | × | × | × | ○ | ○ | ○ | ○ |

**[Table 2]**

| | | Comparative Examples | | | | Examples | | | |
|---|---|---|---|---|---|---|---|---|---|
| | | 1 | 2 | 3 | 4 | 1 | 2 | 3 | 4 |
| Peak 1 | 2θ | 6.1 | none | none | 5.6 | 5.8 | 6.6 | 1.3 | 7.5 |
| | cps | 241.2 | - | - | 696.8 | 322.0 | 340.9 | 449.8 | 876.8 |
| Peak 2 | 2θ | none | 13.8 | none | 16.1 | 15.6 | 15.5 | 15.2 | 14.9 |
| | cps | - | 746.7 | - | 102.9 | 65.2 | 91.9 | 95.6 | 130.0 |
| Peak 3 | 2θ | 26.3 | 24.9 | 25.3 | 26.1 | 26.2 | 26.2 | 26.1 | 26.3 |
| | cps | 94.4 | 214.4 | 152.0 | 165.1 | 80.9 | 106.5 | 109.4 | 101.5 |
| Peak 4 | 2θ | none | 27.0 | 27.4 | 30.0 | 30.1 | 30.4 | 30.3 | 29.4 |
| | cps | - | 143.6 | 209.4 | 99.7 | 63.8 | 71.7 | 80.2 | 69.0 |
| Peak 5 | 2θ | 48.6 | 48.2 | 48.0 | 48.6 | 48.4 | 48.6 | 48.7 | 48.6 |
| | cps | 57.5 | 70.2 | 51.4 | 157.1 | 57.9 | 68.9 | 68.7 | 81.5 |
| Maximum peak at 26°±3° (Peak M) | 2θ | 26.3 | 24.9 | 27.4 | 26.1 | 26.2 | 26.2 | 26.1 | 26.3 |
| | cps | 94.4 | 214.4 | 209.4 | 165.1 | 80.9 | 106.5 | 109.4 | 101.5 |
| Peak 1 / Peak M | | 2.55 | - | - | 4.22 | 3.98 | 3.20 | 4.11 | 8.64 |
| Peak 2 / Peak M | | - | 3.48 | - | 0.62 | 0.81 | 0.86 | 0.87 | 1.28 |

### (Consideration)

All of the titanate aqueous solutions (samples) obtained in Examples 1 to 4 had a light transmittance at a wavelength of 450nm being 100%, and were confirmed as aqueous solutions.

All of the titanate aqueous solutions (samples) obtained in Examples 1 to 4 also had a yellowish tint, and had a light transmittance at a wavelength of 360 nm being 50% or less. This was presumed to be colored by Ti₃O₇²⁻.

It was also found that each of the titanate aqueous solutions (samples) obtained in Examples 1 to 4 could be mixed and reacted with sodium hydroxide or barium hydroxide under normal temperature and pressure to form a sodium or barium titanate compound.

The sodium titanate compound was found to be composed of Na₂Ti₃O₇ hydrate when compared with the XRD pattern described in the above-mentioned reference document.

On the other hand, none of the titanium-containing liquids (samples) obtained in Comparative Examples 1 and 3 were aqueous solutions, and even when mixed with sodium hydroxide under normal temperature and pressure, a sodium titanate compound (Na₂Ti₃O₇ hydrate) could not be obtained.

The titanium-containing liquid (sample) obtained in Comparative Example 2 was an aqueous solution, but contained no quaternary ammonium cations, and even when mixed with sodium hydroxide under normal temperature and pressure, a sodium titanate compound (Na₂Ti₃O₇ hydrate) could not be obtained.

The titanium-containing liquid (sample) obtained in Comparative Example 4 was an aqueous solution, but had a light transmittance at a wavelength of 360 nm being more than 50%, and even when mixed with sodium hydroxide under normal temperature and pressure, a sodium titanate compound (Na₂Ti₃O₇ hydrate) could not be obtained.

Based on the results of the above-mentioned Examples and Comparative Examples, as well as the results of tests conducted by the present inventors so far, it was found that when the primary to tertiary amines were added and reacted in the dissolving step, the liquid containing titanate could not be made into an aqueous solution, whereas when the quaternary ammonium cations were added and reacted, it could be made into an aqueous solution.

Based on the results of the above-mentioned Examples and Comparative Examples, as well as the results of tests conducted by the present inventors so far, it was confirmed that the titanate aqueous solution obtained in each of Examples was reacted with sodium hydroxide to form a precipitate presumed to be Na₂Ti₃O₇ hydrate, and it was thus presumed that the titanate aqueous solution obtained in each of Examples 1 to 4 contained titanate ions and quaternary ammonium cations, and had titanium present in the state of trititanate ions, that is, in the state of (NR₄⁺)₂·Ti₃O₇²⁻.

It was obvious that the titanate aqueous solution (sample) obtained in each of Examples 1 to 4 was an aqueous solution containing no components other than those derived from titanium or titanate and those derived from quaternary ammonium cations, based on the production method. In particular, it was obvious that it contained no hardly-volatile organic components.

Based on the results of the above-mentioned Examples and the results of tests conducted by the present inventors so far, it was considered that when the titanate aqueous solution had peaks present at respective positions of at least 2θ = 6.5° ± 1.4°, 15.5° ± 3°, and 26.0° ± 3° (referred to "peak 1, 2, and 3", respectively), and had an intensity ratio (peak 2/peak M) of the intensity (peak 2 intensity) of the maximum peak at 2θ = 15.5° ± 3° to the intensity (peak M intensity) of the maximum peak at 2θ = 26.0° ± 3° being 0.6 to 2.0, in the X-ray diffraction pattern obtained by subjecting a powder obtained by drying the titanate aqueous solution to X-ray diffraction measurement using CuKα rays, the titanate aqueous solution had high reactivity with hydroxides composed of alkali metals or alkaline earth metals, and when mixed and reacted with the hydroxides under normal temperature and pressure as described above, titanate compounds composed of alkali metals or alkaline earth metals could be easily and reliably obtained.

It was also found that such a titanate aqueous solution preferably had peaks present at 2θ = 30.0° ± 3° and 48.5° ± 3°, respectively.

Furthermore, it was found that such a titanate aqueous solution preferably had an intensity ratio (peak 1/peak M) of the intensity (peak 1 intensity) of the maximum peak at 2θ = 6.5° ± 1.4° to the intensity (peak M intensity) of the maximum peak at 2θ = 26.0° ± 3° being 3.0 or more.

In the X-ray diffraction pattern of Comparative Example 2 in FIG. 8, peaks were present at 2θ = 13.8°, 24.9°, 27.0°, and 48.2°, as shown in Table 2. However, these peaks were not derived from titanium or titanate, but were presumed to be organic substance peaks derived from lactic acid or glycol.

## Claims

1. A titanate aqueous solution comprising titanate ions and quaternary ammonium cations in water,
wherein 30 g of the titanate aqueous solution (25°C) adjusted to a concentration containing 9% by mass of titanium in terms of TiO₂ is added with 30 mL of a sodium hydroxide aqueous solution (25°C) having a concentration of 2.2% by mass while stirring to form a precipitate composed of Na₂Ti₃O₇ hydrate.

2. A titanate aqueous solution comprising titanate ions and quaternary ammonium cations in water,
wherein the titanate aqueous solution has a transmittance at a wavelength of 360 nm being 50% or less.

3. The titanate aqueous solution according to claim 1 or 2, comprising 0.01% by mass to 15% by mass of titanium in terms of TiO₂.

4. The titanate aqueous solution according to any one of claims 1 to 3, having a transmittance at a wavelength of 450 nm being 70% or more.

5. The titanate aqueous solution according to any one of claims 1 to 4, wherein in an X-ray diffraction pattern obtained by subjecting a powder obtained by drying the titanate aqueous solution to powder X-ray diffraction measurement using CuKα rays, the titanate aqueous solution has a ratio of an intensity of the maximum peak at 2θ = 15.5° ± 3° to an intensity of the maximum peak at 2θ = 26.0° ± 3° being 0.6 to 2.0.

6. The titanate aqueous solution according to claim 2, wherein the titanate aqueous solution can be mixed and reacted with aqueous solutions composed of alkali metals or alkaline earth metal salts to obtain titanate compounds composed of alkali metals or alkaline earth metals.

7. The titanate aqueous solution according to claim 6, wherein 30 g of the titanate aqueous solution (25°C) adjusted to a concentration containing 9% by mass of titanium in terms of TiO₂ is added with 30 mL of a sodium hydroxide aqueous solution (25°C) having a concentration of 2.2% by mass while stirring and then stirred for 15 minutes after the addition to form a precipitate composed of Na₄Ti₉O₂₀·nH₂O (n is an integer).

8. The titanate aqueous solution according to any one of claims 1 to 7, wherein the titanate ions comprise Ti₃O₇²⁻.

9. The titanate aqueous solution according to any one of claims 1 to 8, wherein the quaternary ammonium cations comprise cations represented by a general formula of NR₁R₂R₃R₄⁺ (wherein R₁ to R₄ each independently represents a linear, branched-chain, or cyclic hydrocarbon group, alkoxy group, benzoyl group (-COCeHs), or hydroxy group; and all of R₁ to R₄ may be different, all thereof may be the same, or some thereof may be the same).

10. A method for producing a titanate aqueous solution comprising adding a titanium salt solution to an amine aqueous solution to obtain a neutralization reaction liquid (referred to as "neutralization step"), washing a titanium-containing precipitate formed in the neutralization reaction liquid (referred to as "washing step"), and mixing the washed titanium-containing precipitate with a quaternary ammonium salt and water to prepare a titanate aqueous solution (referred to as "dissolving step").

11. The method for producing a titanate aqueous solution according to claim 10, wherein in the dissolving step, a quaternary ammonium salt containing 4 mol or more of quaternary ammonium relative to titanium contained in the washed titanium-containing precipitate is mixed.
